# EUROPEAN PATENT SPECIFICATION

(11) **EP 2 879 630 B1**
(45) Date of publication and mention of the grant of the patent: **28.11.2018**
(21) Application number: 13767114.5
(22) Date of filing: 22.07.2013
(51) Int. Cl.: A61F 5/56

(54) **NEUROPHYSIOLOGICAL STIMULATION DEVICE**
VORRICHTUNG FÜR NEUROPHYSIOLOGISCHE STIMULATION
DISPOSITIF DE STIMULATION NEUROPHYSIOLOGIQUE

(30) Priority: 02.08.2012 IT RM20120380
(43) Date of publication of application: 10.06.2015
(73) Proprietor: Ficacci, Roberta, 00153 Roma (IT)
(72) Inventor: Ficacci, Roberta, 00153 Roma (IT)
(74) Representative: Fiammenghi, Eva
(86) International application number: PCT/IB2013/055997
(87) International publication number: WO 2014/020483

(56) References cited:
- WO-A1-2005/032438
- DE-A1- 10 012 687
- US-A- 5 779 470
- US-A- 5 915 385
- US-A- 6 164 278
- US-A1- 2007 068 534
- US-A1- 2009 120 448

## Description

### Field of the art

The present invention refers to a new and innovative device to be applied in the oral cavity, adapted to induce, in the patient who uses said device, a suitable trigeminal sensory auto-stimulation. This condition is obtained by means of the relaxation of the muscles of the stomatogenetic apparatus by using the present device at regular intervals and for a time period that can vary depending on the symptoms of the disorders affecting the user of the device. The principle on which this approach is based consists of the fact that the human organism is characterized by a fundamental bilaterality. Hence, the result obtained by the use of this device consists of establishing a new functional memory in the organism of the patient, with an ensuing restructuring of the physiological functions that were initially altered due to peripheral asymmetries, like facial asymmetries, contributing to determine a remission of the symptoms or a considerable decrease thereof, e.g. tension at the temporal zone, neck pain, pain at the temporomandibular joint and systemic hyperexcitability.

### State of the art

As is known, all living organisms reveal a common natural tendency upon attaining an internal state of equilibrium. The latter consists of a state of relative stability of the chemical-physical properties of the substances present inside the organisms. The evolution of such properties, in one such state of equilibrium, is commonly called homeostasis and its attainment represents the optimal condition so that all physiological functions, characterizing the living organism, operate correctly and in simultaneous harmony.

In many biological processes, the homeostasis conditions ensure that the chemical concentration of species such as ions and/or molecules is determinate and constant, facilitating the maintenance of the conditions that allow cellular survival.

The human organism is undoubtedly one of the most complex, dynamic living organisms, basically homeostatic and in continuous evolution, whose control is traceable to the plasticity of the nervous system, it too dynamic, adaptive and hence in continuous evolution. Nevertheless, in some cases, and particularly in some subjects, even in the condition in which there is homeostatic equilibrium, disorder states may occur due to functional asymmetries whose presence determines, for example, hypo- and/or hyper-cerebral responses that evolve until fairly serious pathological situations have developed. In other words, even when the various components of the nervous system are initially found in homeostatic conditions, symptoms can occur in the organism that are due to the presence of peripheral asymmetries, such as facial or postural asymmetries, which can cause an altered functioning of neural activity, in the most extreme cases inducing the development of actual pathologies.

It has been demonstrated that asymmetries such as facial asymmetries can influence the trigeminus, cranial nerve V, defined by scientist Antonio Damasio as the "global nerve", given that it is the crossroads of various types of sensory signals afferent to the brain. The close correlation existing between the aforesaid nerve and the facial (as well as postural) asymmetries has therefore led to the idea that therapeutic treatment of the asymmetries would induce a modulation of the disturbing afferents, with a consequent improvement of the neural activity.

This innovative therapeutic approach has its origins in the studies of the Russian scientist I. P. Pavlov, who elaborated the theory of *conditioned reflexes.*

According to the aforesaid theory, there exists a precise bond between an external factor (as a non-limiting example, an acoustic stimulus) and a reaction of the organism. Nevertheless, this type of bond cannot be permanent, but rather temporary. The principles underlying the theory of the conditioned reflexes have led to defining, according to the new scientific acquisitions, this non-declarative learning process as "ASSOCIATIVE LEARNING". Another type of non-declarative learning is that of non-associative learning studied by the scientist E. Thorndike, in which the subject learns the properties of a single stimulus, for example a loud noise, when he is repeatedly exposed to the stimulus and a response is triggered termed tolerance (reduction of the synaptic force) or sensitization (increase of the synaptic force). Tolerance and sensitization are therefore examples of NON-ASSOCIATIVE LEARNING, which are also found in the physiological mechanisms that regulate homeostasis.

As stated above, homeostatic equilibrium conditions are those in which all the physiological functions are carried out in an optimal manner, so that an unbalancing of the equilibrium condition of the NS, induced by peripheral asymmetries, can determine an alteration of the neuronal functions; for example, hypo-functioning areas and hyper-functioning areas are generated such to cause grave diseases, by means of sensitization mechanisms that have initiated. Sensitization is a form of non-associative learning, based on an increase of the synaptic force, in opposition to the concept that tolerance leads to a reduction of the synaptic force (Kandel, Nobel Prize in 2000 for his research on the physiological bases of the preservation of neuron memory).

These considerations led to the birth of a therapeutic approach, which can be defined sensitizing therapy, aiming to modulate the important budget afferent to the cranial nerve V in the clinical conditions characterized by the presence of peripheral asymmetries such as facial asymmetries.

Such nerve therefore has an important role in the neurophysiological equilibrium of the organism. It is called trigeminal nerve because it is formed by three main peripheral branches: the ophthalmic nerve, the maxillary nerve and the mandibular nerve which exit from the cranium through the same number of different holes, i.e. the upper orbital slit, the rotund hole and the oval hole. Underlying the organization of the trigeminal system, there is the general principle according to which the signals directed away from the motors and the information concerning the various sensitive modes are processed in separate nuclei from the trunk of the encephalon. The trigeminal nerve then carries, from peripheral zones to the Central Nervous System, the information necessary for constructing the neuronal cartography, which represents the organism in its entirety. The information transmitted to the CNS subsequently enters into a storage circuit by mean of the limbic system (hippocampus and amygdala), before then being used by the brain to produce vegetative reactions, in order to process motor actions or for constructing mental representations.

The study of the functional asymmetries has thus allowed evaluating that the reorganization of a peripheral function, obtained for the adaptation to a new situation, induces a reorganization of specific functions of the human physiological system, given that the latter is an adaptive, dynamic system and that it tends to respond to the external stimuli. The outside environment is in fact a source of stimuli that are picked up by specific receptors and conveyed by the latter in the form of pulses to the subcortical areas, where they are subjected to a first decoding in order to then be recoded in a language comprehensible to the subsequent units of the system. Such information, passing through different pathways and stations, is sent to the cortex. From here, the perceived signals are sent to the structures assigned for the sensory memory, which thus has the possibility to recognize them. The effector impulses depart from here for the regulation of the homeostatic mechanisms, which have the task of maintaining in equilibrium all the biochemical and biophysical parameters of the internal environment. Therefore, an impulse afferent to the encephalon coming from a peripheral structure that functions in an anomalous manner can cause an afference disturbing the CNS, which in turn stores it, by means of adaptation phenomena, consequently determining the point of departure of a conditioned reflex. For example, if the muscles of one side of the organism are hypotonic or hypertonic, their receptor will send to the CNS a series of impulses with amplitude and frequency such to generate a response proportional to the starting signal. The repetition of the disturbed afferences to the CNS can finally cause the interruption of the equilibrium of a target member, leading the latter to respond in an anomalous manner.

In light of the aforesaid observations and considerations, there arose the intuition to identify a therapeutic approach adapted to modulate and correct the irregularities sometimes characterizing specific peripheral structures, in order to avoid the onset of corresponding disorders, by acting on the functional asymmetries, often responsible for the interruption of the homeostatic equilibria. An innovative device that up to now has allowed reorganizing, by means of adaptation and storage phenomena, physiological functions by correcting asymmetries such as facial asymmetries, is a particular type of bite called "neurological bite". This device described in the patent application ITRM20080141 addresses the symptoms, resolves the pathological situation that initiated, by modulating the intensity of the activity of the specific pathology. For such purpose, it is important to point out that a typical example of structures determining the pathology and activated by a peripheral asymmetry like facial asymmetry is represented by the increase of production of neurotransmitters and/or neuromodulators. For example, from the accumulation of noradrenaline in the encephalon nucleus *locus ceruleus.*

The noradrenaline neurotransmitter constitutes about 1% of the neurotransmitters present at the brain and its hypothesized function is to influence the nervous excitability levels, e.g. by maintaining the latter basal.

A dysfunction of the *locus ceruleus,* induced by an incorrect functioning of a peripheral system, is generally manifested with a hyperactivity of the structure during the REM sleep phase, when this small nucleus should be silent. The fact that it is not silent is due to the continuous request for noradrenaline, whose production is peripherally induced by a periodontal stimulation mechanism. In clenching or gnashing teeth due to stressor situations, an energy production mechanism is thus induced which in a phase like that of sleep should not be requested. Hence, it is clear that by opposing these phenomena with means or devices that prevent the onset of mechanisms that are not necessary for the organism, one contributes to an improvement of the activity of the physiological functions thereof. For example, by inhibiting the production of noradrenaline during the REM sleep phase, the production of serotonin is facilitated; serotonin is another neurotransmitter involved in phenomena such as sleep, body temperature regulation and generally not involving the existence of the organism's nerves in an excited state.

The device described in the document ITRM20080141 acts in an appropriate manner at the neurophysiological level and more in detail by intervening on the extension of the facial muscles and connected nervous pathways, so that it was defined "neurological bite". This device is substantially constituted by a resin base plate which accompanies the palatal arch up to the soft palate, by a block of resin applied in the front zone of the base plate and extended from one canine tooth to the other, and by a plurality of metallic wires having a part inserted in the resin and an external part to be coupled to the teeth. Said device is capable of producing a balanced sensory auto-stimulation by means of the extension of the facial muscles and the nervous pathways connected thereto, which is attained with the insertion in the oral cavity of this particular type of neurophysiological device which acts in a manner such to prevent the closure of the bite and thus send to the sensory stimulation center.

Nevertheless, even if its use is capable of triggering mechanisms that induce a new storage at the neural level and a healing process intended as a re-equilibrium of the dysfunctional situation, this device has limitations. Such limitations substantially consist of the structural characteristics of the device, which are relatively complex, and its mode of application in the oral cavity, for which particular precision is required. For example, the metallic wires composing the device of the document ITRM20080141 must be correctly fixed to the teeth, in particular at least two rows must be fixed to the upper molars. These operations therefore require great accuracy adapted to define a correct functioning of the apparatus. The latter, given its relative structural complexity, can also in some cases and for some patients be irritating, and also cannot be used in contexts involving public relations. This apparatus is indeed only to be used during the night. Characteristics and limits analogous to those described up to now are also represented by other types of dental bites, usually used for preventing bruxism phenomena and which have the common characteristic of being extended over the entire dental arch. The conventional bites, sometimes defined as mandibular positioning plates, are generally applied on the teeth of the lower or upper arch and have the function of preventing the upper and lower molars and premolars from coming into contact with each other in the chewing and swallowing phases, leaving the mandibular condyles free to be arranged where they must naturally be arranged in the swallowing phases, without the interference of a malocclusion. At the current state of the art, the application of the devices obtained up to now is not always capable of reaching therapeutic success, consisting of a correct physiological relationship between the temporomandibular articulation and the dental occlusion. A kind of bite that can be considered most pertinent to the present invention is described in document US 5 779 470 A that discloses a tongue thrust habit oral corrective device designed to be retained by the posterior teeth and to train the tongue to avoid thrusting against the front teeth during swallowing. It is important to noteworthy that an alteration of dental occlusion can also contribute to the establishment of incorrect posture, subsequently giving rise to all of the fairly serious consequences that derive therefrom. For such purpose, the present industrial invention patent application, which will be described in detail hereinbelow, proposes a device having not only characteristics adapted to induce the correction mechanisms of the physiological functions altered by facial peripheral asymmetries or by an incorrect functioning of the peripheral systems, but also having a conformation such to allow the user of the device to be able to apply and remove it to/from the mouth with great simplicity. The object of the present invention, contrary to the conventional bites, only involves the front teeth, placing the premolars and molars at rest.

The present device is also provided with optional additional components capable of also inducing a palatal stimulation that can further modulate the activity of the hypothalamus.

The latter is constituted by a grouping of small nuclei which can be situated on the lower portion of the cerebral mass at the junction between the mesencephalon and the thalamus.

These nuclei are indirectly connected to the palate since they are situated along the base of the encephalon, which is placed just above the palate. The nuclei constituting the hypothalamus are also adjacent to the hypophysis, i.e. the most important of the endocrine glands. The function of the hypothalamus is to integrate the visceral and somatic responses in a manner in accordance with the needs of the brain. The hypothalamus is the coordinator center of the emotions, so that a stimulation carried out at a particular point of the hypothalamus, as well as in several regions of the limbic system, can cause an outburst of anger and/or aggressive behaviour in humans and in some animals. The stimulation of a hypothalamic portion adjacent to the aforesaid point instead causes a sensation of intense pleasure. A great number of receptors are also present in the palate, such receptors also involved in the postural information. More in detail, the palatine stimulation of the tongue is capable of influencing the primary postural receptors from the eye to the foot, the vestibular apparatus and the mandible. Many studies have also appreciated the general muscle relaxant effect and the rebalancing effect on the musculature of the entire body, determined by only palatal stimulation. The stimulation of the palate by the tongue, which is also innervated by the trigeminal nerve, determines various physiological responses that involve the Central Nervous System and the *locus ceruleus.* In particular, the tongue, by compressing the palatine receptors, is capable of indirectly inducing a refunctionalizing of the Central Nervous System. The application of these theories has also allowed providing an explanation of the finger-sucking habits of children older than 2-3 years old.

After this age, indeed, the habit acquired by the child could be linked to the incorrect rest posture of the tongue, which - by not resting at the palatine spot - does not impart the correct stimulus to the trigeminal receptors and the child compensates such deficiency by positioning the finger in the place of the tongue. It was observed that the absence of trigeminal stimulation by the tongue, poorly imitated by the finger, is often accompanied by behavioral disorders in children as well as, in some cases, learning disabilities and emotional disorders.

### Description of the invention

The present invention refers to a new and innovative device adapted to induce a physiological sensory stimulation by acting on the stomatognathic apparatus by means of the extension and relaxation of the facial muscles. The object of the present description is adapted to both remodel the neural activity, initially altered due to peripheral asymmetries, such as facial asymmetries, and preventing phenomena - and more in detail parafunctions, i.e. unintentional activities of the mouth which often occur during sleep, such as clenching teeth, bruxism (gnashing), or biting the tongue, due to stressor conditions of the individual - from altering the correct functioning of the neural activity. These unintentional movements indeed initiate a periodontal mechanism that involves an increase of the quantity of noradrenaline produced, consequently leading the individual to a state of nervous excitation during sleep. The latter condition determines an altered, non-physiological quality of the sleep.

The correct positioning of the device, object of the present patent application, between the two upper and lower dental arches, inevitably causes a decrease of the neuromuscular tension, correctly modifying the production of the noradrenaline neurotransmitter. Said neurophysiological stimulation device is shaped in a manner such to prevent the clenching of the mouth, contrary to what occurs with conventional devices whose use allows the teeth to be in relative contact with each other.

As stated above, the application of the present device is also adapted to modulate and correct the effects of an incorrect functional activity induced by peripheral imperfections, such as the asymmetries commonly diffused at the face. The present device is for example capable of inducing, reflexively, a more equal distribution of the body weight, as well as a correct posture, on the supports of the lower limbs of the individual. This effect is encountered even a few minutes after the positioning of the neurophysiological stimulation device between the dental arches. This occurs due to the close correlation existing between the cranial-sacral system and the cranial-mandibular system. Hence, a dysfunction of the latter induced by a facial asymmetry inevitably involves an incorrect functioning of the cranial-sacral system. The extent of the malfunctioning induced varies according to the extent of the base asymmetry and can be fairly serious, up to becoming pathological. For all applications thereof, the neurophysiological stimulation device, object of the present invention, acts by modulating the information transmitted by the trigeminal nerve, i.e. by the cranial nerve V, to the Central Nervous System, inducing a new storage of the transmitted information and involving a correct construction of a new neuronal mapping that continues even at the end of the treatment.

At the macroscopic level, the application of the device in question in the oral cavity allows obtaining an extension of the facial muscles, existing for the entire positioning time, until a relaxation sensation is induced in the subject, following the sensory auto-stimulation established with the opening of the mouth, which then comes to occlude on the device in question.

A fundamental characteristic of the device, object of the present invention, is that in all the variants or embodiments thereof it has a portion, defined heel, having an incision which is only extended from one canine tooth to the other; hence, both the molars and premolars are left free. Said device is also provided with components adapted to localize the sensory stimulation in precise points, for example at the palate. The intent is to stimulate the palate until a modulatable hypothalamic activity is induced, as well as a correct posture. The palate indeed comprises a region very rich in exteroceptors involved in postural information. More in detail, in a particular embodiment that will be described hereinbelow, the present device has a component adapted to simulate the correct pressure exerted by the tongue against the palate, encountering beneficial effects on the musculature that are extremely clear and immediate. More in detail, this component allows correcting aspects such as muscular hypertonia and rebalancing the tone itself in an instantaneous manner. These effects can be encountered on muscles such as the stomatognathic muscles (anterior temporal, masseter, anterior digastric), some trunk muscles (para-vertebral, latissimus dorsi) and the muscles of the lower limbs. Also in this case, the pathologies caused by a functional imbalance of the aforesaid muscles can be correlated with an insufficient trigeminal stimulation, in the specific case providing an explanation for the scoliotic posture as a consequence of an insufficient stimulation of the palate receptors.

The present device is also optionally provided with components and more in detail with substances that amplify the stimulation, by acting on the olfactory system and on the stomatognathic system.

More in detail, the present device optionally includes the presence of substances that guide the relaxation by acting on the limbic system, i.e. on the deepest part of the brain that is responsible for the control of the emotions and moods and which is closely connected with the olfactory terminations.

The described neurophysiological stimulation device is therefore optionally provided with a system for releasing substances orally or for supplying fragrances of substances such as active principles or phytotherapeutic products for relaxation or calming purposes. This leads to the induction of the release of endorphins and serotonin in the subject, bringing the latter into a state of induced relaxation. As will be described in detail hereinbelow, the present device is also optionally provided with components adapted to make the mechanism for sensory stimulation more effective. In particular, the device according to the present invention is optionally provided with common lingual stimulators and/or with substances with various flavors acting on the lingual and olfactory receptors.

The duration of the positioning of the described device varies from subject to subject and can occur for a few minutes in the course of the day, or before the subject goes to sleep, or upon waking at night, until the induced relaxation is obtained.

### Brief description of the drawings

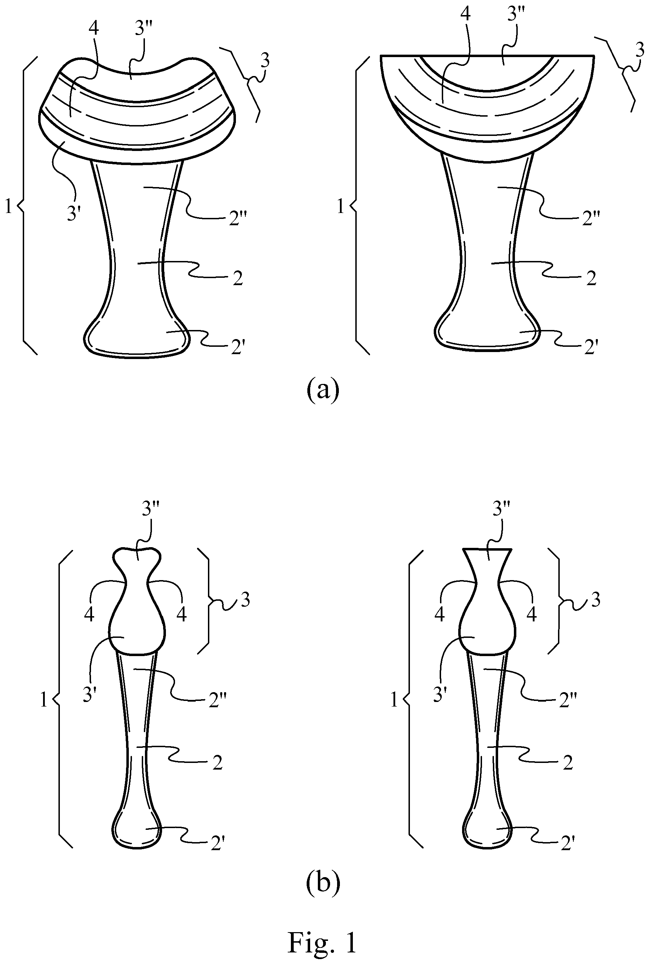

FIGURE 1 shows a front and lateral face of the device 1 for neurophysiological stimulation induced by facial relaxation and object of the present description. More in detail, figure 1(a) shows a front view of the device 1 in which the anvil portion 2 and the curved portion 3 can be observed. In particular, the portion 2, adapted to constitute the grip of the device 1 and having an anvil-shaped profile, has two ends, i.e. the first end 2' and the second end 2", the latter firmly connected to the curved portion 3. As is observed from the figure, the curved portion 3, it too having two ends i.e. the convex end 3' and the flat or concave end 3", is extended perpendicular to the anvil portion 2 with which said curved portion 3 is connected. More in detail, the convex end 3' of the curved portion 3 is joined with the second end 2" of the anvil portion 2. The curved portion 3, profiled as an arc and hence curved, also has an incision 4 on its surfaces, such incision following the curvature of the curved portion 3, and adapted to represent the dental occlusion zone which is extended from one canine tooth to the other. Said curved portion 4- 3 is more in detail delimited by the convex end 3' and by the flat or concave end 3". The latter, i.e. said flat or concave end 3", can therefore be concave or flat. Figure 1 (b) instead shows a side view of the device 1 in which all the characteristics and components described in figure 1(a) can be encountered. The figure also shows the particular profile of the flat or concave end 3" which in one case is concave and in the other is flat.

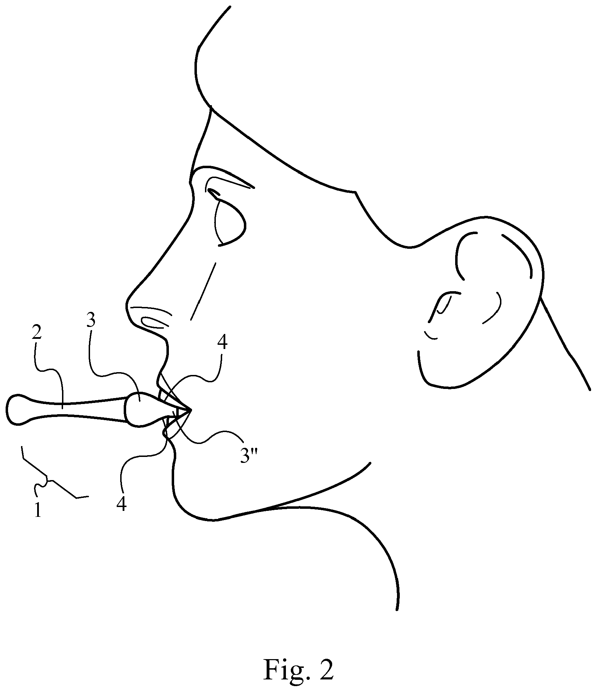

FIGURE 2 shows a side view of a subject user of the device 1 in which the mode is observable in which said device 1 is inserted in the oral cavity of the patient. It is observed from the figure that the anvil portion 2 along with part of the curved portion 3 are extended outside the mouth, while the incision 4 is involved in the dental bite from one canine tooth to the other of the patient and the flat or concave end 3" is extended inside the mouth beyond the dental arch.

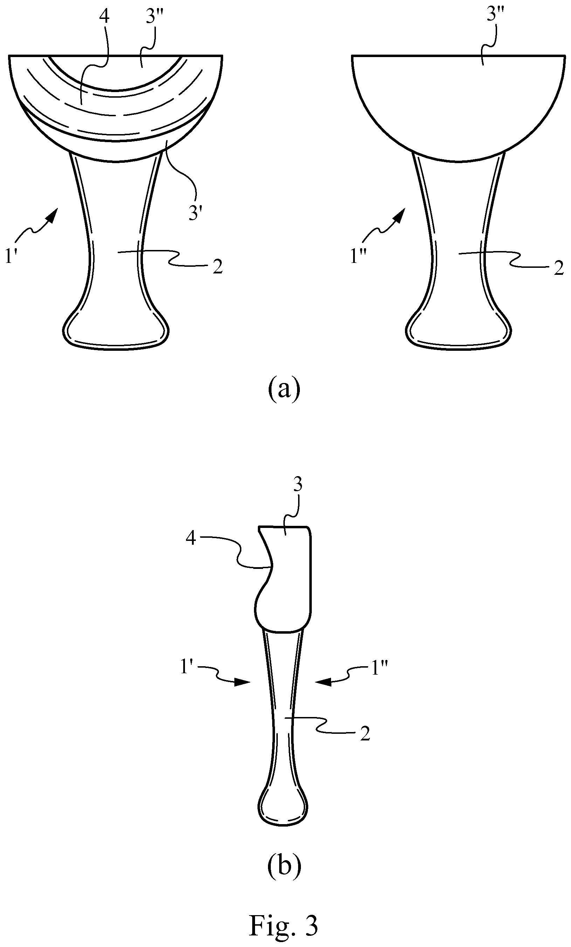

FIGURE 3 shows a front and side view of the neurophysiological stimulation device 1 in a particular embodiment thereof. More in detail, figure 3(a) shows a front view of both surfaces of the device 1, i.e. the first surface 1' and the second surface 1". The figure shows the presence of the incision 4 only on one of the two surfaces and in particular said incision 4 preferably is localized on the surface adapted to involve, in the dental bite, the canine teeth of the upper dental arch, leaving those of the lower arch freer to move, which will come to be positioned on the smooth surface of the curved portion 3 opposite the surface having said incision 4.

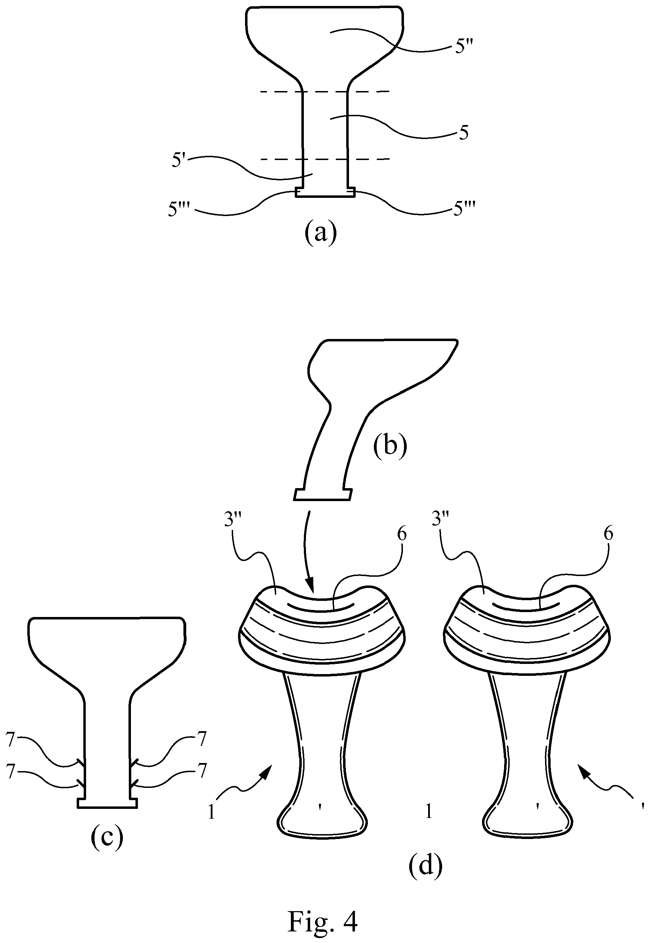

FIGURE 4 shows a front perspective view of the device 1 in a particular embodiment thereof according to the present invention. More in detail, figure 4(a) shows a front view of the palatal element 5 to be assembled to the device 1 and adapted to induce stimulation via trigeminal nerve of the receptors of the palate in order to induce postural and emotional corrections in the patient. More in detail, in figure 4(a) it is observed that said palatal element 5 has two ends, i.e. a first end of palatal element 5' and a second end of palatal element 5", the latter having a profile with palatal arch, and adapted to stimulate the front portion of the palate of the subject user of the device 1. The first end of palatal element 5' instead has small lateral projections 5''' adapted to allow the insertion and locking of said palatal element 5 with the device 1. Figure 4(b) instead shows a perspective view of said palatal element 5 in which its curved conformation and arc-shaped profile can be observed. Figure 4(c) shows a front view of a particular embodiment according to the present invention, in which said palatal element 5 is optionally provided with at least one pair of flexible tabs 7 that are extended along the edges of the greater sides of said palatal element 5. Said flexible tabs 7 have the function of adjusting the extension of the palatal element 5 along the palate of the patient according to the depth of the oral cavity of the subject. Figure 4(d) shows a front view of the two surfaces of the device 1, object of the invention, i.e. a front view of the first surface 1' and of the second surface 1". The object of the figure is to underline the presence of a slit 6 at the flat or concave end 3" of the curved portion 3, such slit adapted to allow the passage of the palatal element 5 from the surface 1'(or 1") to the other, in this manner facilitating the assembly and coupling of said palatal element 5 to the device 1.

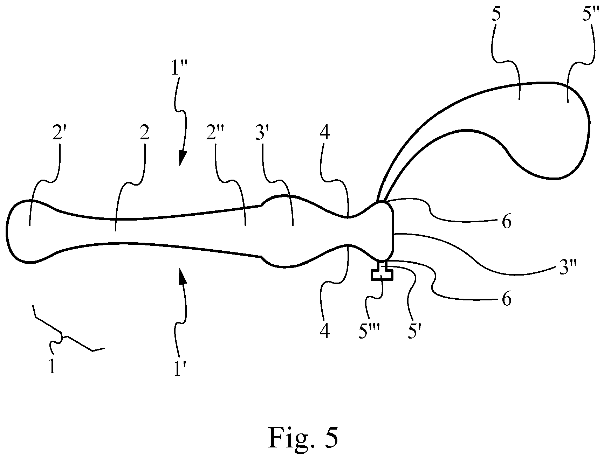

FIGURE 5 shows a side view of the device 1 when the palatal element 5 is assembled thereto. As is observed in the figure, where furthermore all the components described in the preceding figure are visible, the palatal element 5 is spatially arranged in a manner such to prevent the same from accidentally falling in the oral cavity. The presence of the lateral projections 5''' situated at the base of the first end of the palatal element 5' confers a length to the latter greater than the slit 6, preventing the unintentional exit from said slit 6 by the element 5. Said lateral projections 5''' are indeed locked on the surface of the device 1.

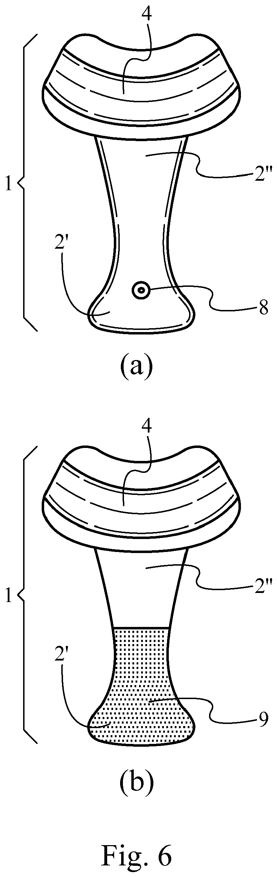

FIGURE 6 shows a front view of the neurophysiological stimulation device 1 in a particular embodiment according to the present invention. More in detail, figure 6(a) shows a front view of the device 1 in which a nebulizing spray hole 8 is observable at the anvil portion 2, such hole adapted to allow the delivery of the substance, preferably phytotherapeutic, or of active principles, contained inside said anvil portion 2, given that the latter is made internally hollow in this case. The delivery occurs by manually compressing said anvil portion 2 which in this embodiment is preferably made of an amorphous polymer that can be easily manually deformed. Figure 5(b) instead shows a front view of the device 1; here, on part of the surface of the anvil portion 2, a membrane 9 is observable adapted to retain the phytotherapeutic substance on its surface by means of adsorption phenomena, in a manner such that the fragrance emanated from said substance can be easily picked up by the olfactory receptors of the subject user of the device 1.

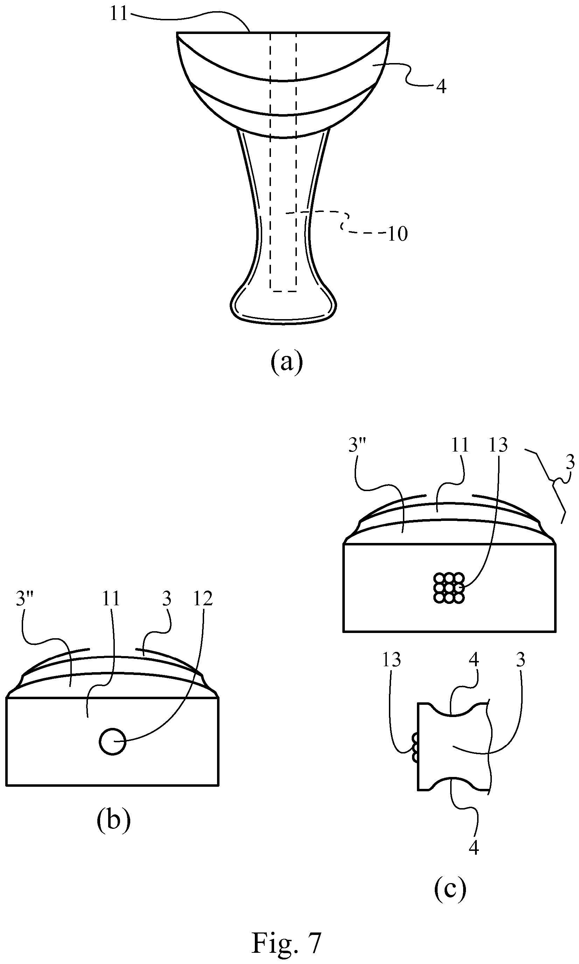

FIGURE 7 shows a front and perspective view of particular embodiments of the neurophysiological stimulation device 1 according to the present invention. More in detail, figure 7(a) shows a front view of the device 1 when at its interior and in a longitudinal direction thereto, from the anvil portion 2 to the curved portion 3, there is the channel 10 adapted to contain active principles or phytotherapeutic substances to be directly and orally administered to the subject. More in detail, the release of the substance inside the mouth of the subject occurs due to the presence of the hole 12 placed on the surface 11 of the portion 3, as is observed in figure 7(b). The substance is released by manually compressing the deformable anvil portion 2, and hence by indirectly compressing also the channel 10 containing the fluid that will exit from said hole 12. Figure 7(c) shows a perspective and side view of the curved portion 3 of the device 1 in another embodiment. More in detail, figure 7(c) shows the presence of a lingual stimulator 13 adapted to enhance the sensory stimulation of the subject. Said element is placed on the surface 11 of the second flat or concave end 3", preferably flat, of the curved portion 3.

### Description of the embodiments

In a first embodiment, the device 1 for neurophysiological stimulation induced by the relaxation of the facial system is represented by a single body on which at least two portions can be identified having different profile and extension. More in detail, the device 1 for sensory stimulation comprises at least one anvil portion 2 profiled as an anvil to be placed outside the mouth and adapted to constitute the grip of said device 1, and at least one curved portion 3, which is extended perpendicular to said anvil portion 2 with which said curved portion 3 is directly connected. More in detail the anvil portion 2, profiled as an anvil, has an a first end 2' extended towards the exterior of the mouth of the subject user of the device 1, and a second end 2" connected with the curved portion 3 and still more in detail with the convex end 3' of the curved portion 3. The latter indeed has a convex end 3' that is externally extended with respect to the teeth of the subject upon dental occlusion, and a concave or flat end 3" that is internally extended with respect to the teeth of the subject when said teeth occlude on the device 1. The latter furthermore has, on the curved portion 3, an incision 4 which more in detail is delimited by the aforesaid ends 3' and ends 3". The incision 4 follows the curvature of the curved portion 3 and is adapted to constitute the zone in which said device 1 acts on the mouth. In particular, said incision 4 is extended from one canine tooth to the other when the device 1 is applied to the subject user of the same. Said incision 4, in this embodiment, is also present on both first surfaces 1' and second surface 1" of the device and more in detail on both surfaces of the curved portion 3. This characteristic ensures that both the canine teeth of the lower dental arch and those of the upper dental arch are involved in the occlusion mechanism in specific points of the device 1 and in particular in specific points of the incision 4. In this simple but effective embodiment, the neurophysiological stimulation device 1 acts on the subject, inducing the relaxation of the facial muscles in a suitable manner, involving a new flow of signals to the CNS via the trigeminal nerve which is subsequently acquired and stored, continuing even at the end of the stimulation.

As an alternative and in another embodiment of the present device 1, the curved portion 3 has the incision 4 only on one of its surfaces involved in the mouth occlusion. Preferably said incision 4 is present on the surface of the curved portion 3 adapted to occlude the teeth from one canine tooth to the other of the upper dental arch of the subject user of the device 1. This characteristic allows conferring greater freedom of movement to the teeth, from one canine tooth to the other, of the lower dental arch of the subject, given that the surface of the curved portion 3 on which said teeth of the lower arch occlude is smooth. In another embodiment, the neurophysiological stimulation device 1 comprises a palatal element 5 for palatal sensory stimulation, it too acting via the trigeminal nerve. More in detail, said palatal element 5 is adapted to be assembled to the device 1 in a manner so as to induce a suitable stimulation of the exteroceptors present at the palate. This occurs since said component simulates the contact and the pressure that the tongue should exert on the palate in conditions of homeostasis. In particular, said palatal element 5, with suitable rigidity, has the shape of a rigid arc and has a first end of palatal element 5' and a second end of palatal element 5". In particular, the first end of palatal element 5' is slightly enlarged with respect to the central portion of the palatal element 5 since said first end of palatal element 5' has lateral projections 5"'. The second end of palatal element 5" is instead considerably enlarged with respect to the central portion of the palatal element 5, since said second end of palatal element 5" is profiled with palatal arch, i.e. it has a profile similar to and in accordance with that of the anatomic structure of the human palate. The assembly of said palatal element 5 to the device 1 occurs due to the presence of a slit 6 present on the end 3" of the curved portion 3 and adapted to allow the passage of the palatal element 5 from the first surface 1' to the second surface 1" (and/or vice versa) of the device 1. More in detail, when the palatal element 5 is assembled to the device 1 via insertion and locking of the first end of the palatal element 5' through the slit 6 and when said device 1 is applied to the mouth of the patient, the palatal element 5 is spatially configured in a manner such to have the first end of palatal element 5' inside the mouth with respect to the lower dental arch and outside the first surface 1' (or second surface1") of the device 1, whereas the second end of palatal element 5", outside the second surface 1" (or first surface 1'), also extends inside the mouth and towards the upper dental arch in a manner so as to be situated in contact with the front portion of the palate. This assemblage palatal element 5 is also shaped and structured in a manner such to allow its contact with the front portion of the palate and at the same time to prevent the same palatal element 5 from accidentally falling in the oral cavity of the patient or from inducing a vomit reaction. This condition is made impossible due to the presence of the aforesaid lateral projections 5''' adapted to prevent the unintentional disengagement of the palatal element 5 from the device 1. The first end of palatal element 5' indeed has, due to said lateral projections 5"', an overall length that is greater than the length of the slit 6, so that said projections 5'" by impacting with the first surface 1' (or second surface 1") of the device 1 prevent the exit of the first end of palatal element 5' from the slit 6. The same lateral projections 5''' are in any case such to allow the manual insertion and coupling of the first end of palatal element 5' through the slit 6, by exerting a relatively small force. This characteristic allows the user to very easily assemble and disengage the palatal element 5 to/from device 1 and at the same time prevent the same palatal element 5 from accidentally separating from said device 1 during the use thereof. The palatal element 5, in another embodiment, can be optionally provided with a plurality and more in detail with at least one pair of flexible tabs 7, which are arranged along the edges of the greater sides thereof.

The pairs of flexible tabs 7 have the function of allowing the adjustment of the palatal element 5 inside the mouth of the patient. More in detail, the pairs of tabs 7 allow pushing the palatal element 5 along the palate to a specific depth and locking it at a specific point of the latter. These components, i.e. the pairs of tabs 7, thus allow taking under consideration the length and the depth of the oral cavity, which can vary from subject to subject.

In another example not falling under the scope of the present invention, the device 1 is obtained in a manner such to have the palate stimulation element 5 already integrated with said device 1. More in detail, in this particular example the element 5 is irreversibly and firmly connected, in proximity to its first end of palatal element 5', with the flat or concave end 3" of the curved portion 3.

In yet another embodiment, the neurophysiological stimulation device 1 that is the object of the present description is provided with systems that allow the delivery and/or release of the substances such as, as a non-limiting example, active principles or substances with phytotherapeutic action, whose fragrance induces the release of relaxing substances such as endorphins starting from the olfactory system. More in detail, in order to have this particular characteristic in the present device 1, the latter is obtained in a manner such to have the curved portion 2 internally hollow. This allows being able to insert, by means of common operations and means of known type (e.g. by means of injection), the substances with relaxing action inside said anvil portion 2. Said substance will be easily delivered by the device 1 due to the presence of at least one common nebulizing spray hole 8, or of an equivalent delivery system, preferably placed in proximity to the first end 2' of the device 1. The anvil portion 2 of the device, in this embodiment internally hollow, is furthermore made of an amorphous polymer that is easily deformed when manually compressed. The mechanical compression action of the anvil portion 2, applied by the user of the device 1, thus allows the exit of the substance in the nebulized state from the hole 8; the fragrance of the substance will cause the aforesaid induction mechanisms for the relaxation of the subject who uses the device 1.

For the same purpose, and in another embodiment, the neurophysiological stimulation device 1 is provided on the surface of the anvil portion 2 with a membrane 9 of adsorbent material ideal for phytotherapeutic substances with relaxing action and/or for specific active principles. More in detail, the presence and composition of the membrane 9 is such to define the chemical-physical interactions between the molecules that constitute it and those of the phytotherapeutic substance. The type of chemical-physical interaction that occurs is that which usually intervenes in adsorption phenomena i.e. electrostatic interactions, Van der Waals interactions, hydrogen bonds and other similar, relatively weak interactions. The presence of the membrane 9, in this embodiment, thus allows retaining the substance on its surface, by previously immersing the anvil portion 2 in a phytotherapeutic fluid or in a fluid containing the active principle. In this manner, the fragrance of the adsorbed substance is localized in the spatial region that surrounds the anvil portion 2 and hence in proximity to the olfactory receptors of the subject user of the device 1.

In another embodiment, the device 1 is structured in a manner such to allow the direct release of the phytotherapeutic substance or the active principle orally. More in detail, in this embodiment the device 1 is provided with an internal channel 10, which is extended longitudinally and inside the device 1 from the anvil portion 2 to the curved portion 3 and more in detail up to the surface 11 of the flat or concave end 3". On said surface 11, which can be flat or concave, a hole 12 is also placed adapted for the outflow of the substance. The delivery of the substance occurs by manually and laterally compressing the anvil portion 2, inducing the passage of the liquid inside the channel 10, from said channel to the outlet hole 12, and consequently inside the mouth of the subject user of the device 1. The introduction of the phytotherapeutic substances or active principle inside said channel 10 occurs by executing common operations and by employing devices of known type, e.g. the introduction of the substance can occur via injection of the liquid in points of the anvil portion 2 corresponding to said channel 10.

In a further embodiment the device 1, object of the present invention, is also optionally provided with common lingual stimulators and in particular with at least one common lingual stimulator 13, represented by an agglomerate of spherules, preferably placed on the surface 11 of the curved portion 3. Furthermore, the present device 1 in another embodiment is provided with sensory stimulators and in particular with taste stimulators consisting of a specific flavor that is imparted to said device 1 and preferably to its curved portion 3. Hence, in this embodiment, said device 1 can have various flavors, e.g. fruity flavors, that stimulate the mouth of the subject, making the entire process of neurophysiological stimulation induced by the device 1 more efficient overall.

In all embodiments thereof, the neurophysiological stimulation device 1 described in the present invention is obtained of a polymer material such as acrylic resin, silicone and the like.

In addition, the length of the anvil portion 2, measured from the edges of the first end 2' to those of the second end 2", is comprised between 2 cm and 6 cm and preferably is 3 cm. The width of said anvil portion 2 instead varies between 0.2 cm and 3 cm and preferably is 2 cm at the first end 2' and the second end 2" and preferably is 1.5 cm in the central zone and its thickness is comprised between 0.3 cm and 1 cm and preferably is 0.4 cm in the central zone and 0.7 cm at the end 2' and the end 2". The curved portion 3 has a thickness in proximity to the convex end 3' and flat or concave 3" comprised between 0.2 cm and 3 cm and preferably is 1.5 cm, said curved portion 3, when its flat or concave end 3" is concave, furthermore has at said flat or concave end 3" a radius of curvature that is comprised between 0.5 cm and 3 cm and preferably is 1 cm. Similarly, the convex end 3' has a radius of curvature comprised between 0.5 cm and 3 cm and preferably is 2 cm. The incision 4 has a depth that varies between 0.1 cm and 0.8 cm and preferably is 0.3 cm. The optionally-added palatal element 5 for palatal sensory stimulation, previously integrated with the device 1, has a length comprised between 3 cm and 7 cm and preferably is 3.5 cm from the first end of palatal element 5' to the second end of palatal element 5" and it has, given that it is curved as an arc, a radius of curvature whose value is preferably comprised between 1 cm and 3 cm and preferably is 2 cm; furthermore, the width of said palatal element 5 is comprised between 1 cm and 6 cm and preferably is 2 cm at the lateral projections 5''' placed at the first end of palatal element 5' whereas it is preferably 4 cm at the second end of palatal element 5".

## Claims

1. Device (1) for neurophysiological stimulation induced by facial relaxation comprising: an anvil portion (2) profiled as an anvil and having a first end (2') and a second end (2"); a curved portion (3) profiled as an arc and having a convex end (3') and a flat or concave end (3"), an incision (4) present on said curved portion (3) following the same curvature as the latter, said anvil portion (2) and the curved portion (3) forming a single body, said anvil portion (2) and said curved portion (3) being firmly joined together by having said second end (2") of the anvil portion (2) joined with said convex end (3') of the curved portion (3), said curved portion (3) being adapted to represent the occlusion zone of the bite of the user, said curved portion (3) having the incision (4) extended from one canine tooth to the other at the time of the application of the device (1) to the subject user of the device (1), the flat or concave end (3") extended inside the mouth with respect to the teeth of the subject and the convex end (3') extended outside the teeth of the user, said incision (4) being delimited by said convex end (3') and by said flat or concave end (3"), said incision (4) being present on at least one of the first surface (1') and/or second surface (1") of the device (1) and preferably on at least the surface involving the dental occlusion of the canine teeth of the upper dental arch, and said anvil portion (2) being adapted to represent the grip of the device (1), said anvil portion (2) being extended outside the mouth of the user at the time of its application, said device (1) being **characterized in that** it comprises a palatal element (5), having a first end of the palatal element (5') and a second end of the palatal element (5"), said palatal element (5) being adapted for sensory stimulation of the arch of the front palate, said palatal element (5) having said second end of the palatal element end (5"), in use, in engagement with the palatal arch, and said palatal element (5) being such to be assembled and reversibly locked to said device (1) at its curved portion (3), said curved portion (3) having the slit (6) on its flat or concave end (3"), such slit being adapted to allow the passage of said palatal element (5) from one surface of the device (1) to the other, said device (1) having the first surface (1') and the second surface (1") and said palatal element (5) having the first end of the palatal element (5') with lateral projections (5"') adapted to allow the manual insertion and locking of the palatal element (5) through said slit (6) and at the same time adapted to prevent the unintentional disengagement of said palatal element (5) from the device (1), said lateral projections (5"') conferring a greater width to the first end of the palatal element (5') with respect to that of the slit (6).

2. Device (1) for neurophysiological stimulation induced by facial relaxation according to the preceding claim, **characterized in that** the palatal element (5) for sensory stimulation of the palate further comprises at least one pair of flexible tabs (7) placed along the greater sides of said palatal element (5) that are adapted to adjust the extension of said palatal element (5) along the palate of the subject user of the device (1).

3. Neurophysiological stimulation device (1) according to the preceding claims, **characterized in that** it further comprises systems for delivering active principles or substances with phytotherapeutic action, said device (1) having the anvil portion (2) internally hollow and adapted to contain the phytotherapeutic substance, and at least one nebulizing spray hole (8) preferably in proximity to the first end (2') of said anvil portion (2) and adapted for the outflow of the substance, said anvil portion (2) being made of an amorphous polymer that is manually compressible, and/or **in that** it comprises systems with slow release of active principles or phytotherapeutic substances, said device (1) optionally comprising the membrane (9), adsorbent for active principles and/or for substances with phytotherapeutic action, on the surface of the anvil portion (2) and adapted to retain said substances after having immersed said anvil portion (2) inside a phytotherapeutic fluid, or a fluid containing active principles, and to slowly release said phytotherapeutic substance or active principles in proximity to the olfactory receptors of the subject when he/she uses the device (1).

4. Neurophysiological stimulation device (1) according to the preceding claims, **characterized in that** it further comprises a system for delivering substances and
preferably for the delivery of active principles or phytotherapeutic substances to be directly and orally administered to the subject, said device (1) comprising at least one channel (10) adapted to contain the fluid substance, such channel directed internally and longitudinally with respect to said device (1) from the anvil portion (2) to the curved portion (3), and at least one hole (12) placed on the end (3") of the portion (3), said convex end (3") having the surface (11) on which said hole (12) is preferably placed, and said anvil portion (2) being made of a compressible material, such to induce the indirect compression of said channel (10) containing the fluid to be delivered and previously inserted with means and operations of known type.

5. Neurophysiological stimulation device (1) according to the preceding claims, **characterized in that** it further comprises lingual stimulators, said device (1) comprising at least one lingual stimulator (13), preferably represented by an agglomerate of spherules, preferably to be placed on the surface (11) of the flat or concave end (3") of the curved portion (3).

6. Neurophysiological stimulation device (1) according to the preceding claims, **characterized in that** the anvil portion (2) has a length comprised between 2 cm and 6 cm and preferably is 3 cm, a width comprised between 0.2 cm and 3 cm and preferably is 2 cm in proximity to the first end (2') and the second end (2") and preferably is 1.5 cm in the central zone, a thickness comprised between 0.3 cm and 1 cm and preferably is 0.4 cm in the central zone of said anvil portion (2) and preferably is 0.7 cm at the first end (2') and the second end (2"), **in that** the curved portion (3) has a thickness in proximity to the convex end (3') and to flat or concave end (3") comprised between 0.2 cm and 3 cm and preferably is 1.5 cm, a radius of curvature comprised between 0.5 cm and 1.5 cm and preferably is 1 cm at its flat or concave ends (3") when said flat or concave end (3") is curved, and a radius of curvature comprised between 0.5 cm and 3 cm and preferably is 2 cm at the convex end (3'), and **in that** the incision (4) has a depth that varies between 0.1 cm and 0.8 cm and preferably is 0.3 cm.

7. Neurophysiological stimulation device according to the preceding claims, **characterized in that** it is further obtained with substances adapted to enhance the lingual sensory stimulation, said device (1) being optionally obtained with edible substances conferring various flavors to said device (1) and preferably fruity flavors.

8. Device (1) for neurophysiological stimulation induced by facial relaxation according to the preceding claims, **characterized in that** the palatal element (5) for the palatal sensory stimulation has a length comprised between 3 cm and 7 cm and preferably is 3.5 cm measured from the first end (5') to the second end (5"), a radius of curvature comprised between 1 cm and 3 cm and preferably is 2 cm, and a width comprised between 1 cm and 6 cm and preferably is 2 cm at the lateral projections (5''') placed at the first end of the palatal element (5') and preferably is 4 cm at the second end of the palatal element (5").

## Patentansprüche

1. Vorrichtung (1) für neurophysiologische Stimulation hervorgerufen durch Gesichtsentspannung, die aufweist: einen Ambossteil (2), der als Amboss profiliert ist und ein erstes Ende (2') und ein zweites Ende (2") aufweist; einen gekrümmten Teil (3), der als Bogen profiliert ist und ein konvexes Ende (3') und ein flaches oder konkaves Ende (3"), einen Einschnitt (4), der auf dem gekrümmten Teil (3) vorliegt und dieselbe Krümmung wie letzterer besitzt, aufweist, wobei der genannte Ambossteil (2) und der gekrümmte Teil (3) einen einzigen Körper bilden, wobei der genannte Ambossteil (2) und der genannte gekrümmte Teil (3) fest miteinander verbunden sind, indem das zweite Ende (2") des Ambossteils (2) mit dem genannten konvexen Ende (3') des gekrümmten Teils (3) verbunden ist, wobei der genannte gekrümmte Teil (3) so angepasst ist, dass er die Okklusionszone des Benutzers abbildet, wobei der genannte gekrümmte Teil (3) zur Zeit der Anwendung der Vorrichtung (1) auf den Benutzer der Einrichtung (1) den Einschnitt (4) sich von einem Eckzahn zu dem anderen erstreckend, das flache oder konkave Ende (3") sich in Bezug auf die Zähne des Subjekts im Inneren des Mundes erstreckend und das konvexe Ende (3') sich außerhalb der Zähne des Benutzers erstreckend aufweist, wobei der genannte Einschnitt (4) durch das genannte konvexe Ende (3') und durch das genannte flache oder konkave Ende (3") begrenzt wird, wobei der genannte Einschnitt (4) an zumindest einer von der ersten Oberfläche (1') und/oder zweiten Oberfläche (1") der Vorrichtung (1) und bevorzugt an zumindest der Oberfläche, die die Dentalokklusion der Eckzähne des oberen Zahnbogens zur Folge hat, vorhanden ist, und wobei der genannte Ambossteil (2) dazu ausgebildet ist, den Griff der Vorrichtung (1) darzustellen, wobei sich der genannte Ambossteil (2) zur Zeit seiner Anwendung außerhalb des Mundes des Benutzers erstreckt, wobei die genannte Vorrichtung (1) **dadurch gekennzeichnet ist, dass** sie ein palatales Element (5) mit einem ersten Ende des palatalen Elements (5') und einem zweiten Ende des palatalen Elements (5") aufweist, wobei das genannte palatale Element (5) zur sensorischen Stimulation des Bogens des Frontgaumens angepasst ist, wobei das genannte palatale Element (5) bei der Benutzung das zweite Ende des palatalen Elements (5") im Eingriff mit dem Gaumenbogen aufweist und das genannte palatale Element (5) dergestalt ist, dass es zusammengebaut und an seinem gekrümmten Teil (3) reversibel an der genannten Vorrichtung (1) befestigt werden kann, wobei der gekrümmte Teil (3) den Schlitz (6) an seinem flachen oder konkaven Ende (3") aufweist, wobei ein derartiger Schlitz dazu angepasst ist, den Durchtritt des genannten palatalen Elements (5) von einer Oberfläche der Vorrichtung (1) zu der anderen gestattet, wobei die genannte Vorrichtung (1) die erste Oberfläche (1') und die zweite Oberfläche (1") aufweist und wobei das genannte palatale Element (5) das erste Ende des palatalen Elements (5') mit lateralen Vorsprüngen (5''') aufweist, die dazu angepasst sind, das manuelle Einführen und das Befestigen des palatalen Elements (5) durch den genannten Schlitz (6) zu erlauben und die zugleich dazu angepasst sind, das unbeabsichtigte Ausrücken des genannten palatalen Elements (5) von der Vorrichtung (1) zu verhindern, wobei die genannten lateralen Vorsprünge (5''') dem ersten Ende des palatalen Elements (5') in Bezug auf die des Schlitzes (6) eine größere Breite verleihen.

2. Vorrichtung (1) für neurophysiologische Stimulation hervorgerufen durch Gesichtsentspannung gemäß dem vorangehenden Anspruch, **dadurch gekennzeichnet, dass** das palatale Element (5) zur sensorischen Stimulation des Gaumens weiterhin zumindest ein Paar entlang der größeren Seiten des genannten palatalen Elements (5) angeordneter flexibler Streifen (7) aufweist, die dazu angepasst sind, die Ausdehnung des genannten palatalen Elements (5) entlang des Gaumens des Benutzers der Vorrichtung (1) anzupassen.

3. Neurophysiologische Stimulationsvorrichtung (1) gemäß den vorangehenden Ansprüchen, **dadurch gekennzeichnet, dass** sie weiterhin Systeme zum Abgeben aktiver Wirkstoffe oder Substanzen mit phytotherapeutischer Wirkung aufweist, wobei die genannte Vorrichtung (1) den Ambossteil (2), der innen hohl und dazu, die phytotherapeutische Substanz zu enthalten, ausgebildet ist, aufweist, sowie zumindest ein Zersteubungssprühloch (8), das sich bevorzugt in der Nähe des ersten Endes (2') des genannten Ambossteils (2) befindet und zum Ausströmen der Substanz ausgebildet ist, wobei der genannte Ambossteil (2) aus einem amorphen Polymer, das manuell kompressibel ist, gemacht ist, und/oder dadurch, dass sie Systeme mit langsamer Abgabe aktiver Wirkstoffe oder phytotherapeutischer Substanzen aufweist, wobei die genannte Vorrichtung (1) optional die Membran (9), ein Adsorptionsmittel für aktive Wirkstoffe und/oder für Substanzen mit phytotherapeutischer Wirkung, aufweist, die auf der Oberfläche des Ambossteils (2) angeordnet und dazu ausgebildet ist, die genannten Substanzen zu speichern, nachdem der Ambossteil (2) in ein phytotherapeutisches Fluid oder ein Fluid, das aktive Wirkstoffe enthält, eingetaucht wurde, und die genannte phytotherapeutische Substanz oder die aktiven Wirkstoffe in der Nähe der olfaktorischen Rezeptoren des Benutzers, wenn er/sie die Vorrichtung (1) benutzt, langsam freizugeben.

4. Neurophysiologische Stimulationsvorrichtung (1) gemäß den vorangehenden Ansprüchen, **dadurch gekennzeichnet, dass** sie weiterhin ein System zum Abgeben von Substanzen und bevorzugt zum Abgeben aktiver Wirkstoffe oder phytotherapeutischer Substanzen, die dem Benutzer direkt und oral zu verabreichen sind, aufweist, wobei die genannte Vorrichtung (1) zumindest einen Kanal (10), der dazu ausgebildet ist, die fluide Substanz aufzunehmen, aufweist, wobei ein derartiger Kanal intern und in Bezug auf die genannte Vorrichtung (1) longitudinal von dem Ambossteil (2) zu dem gekrümmten Teil (3) gerichtet ist, und zumindest ein Loch (12), das an dem Ende (3") des Teils (3) platziert ist, wobei das genannte konvexe Ende (3"), das die Oberfläche (11), auf der das genannte Loch (12) bevorzugt platziert ist, aufweist, und wobei der genannte Ambossteil (2) aus einem kompressiblen Material gemacht ist, um die indirekte Kompression des genannten Kanals (10), der das abzugebende und zuvor mit Mitteln und Abläufen bekannten Typs eingebrachte Fluid enthält, hervorzurufen.

5. Neurophysiologische Stimulationsvorrichtung (1) gemäß den vorangehenden Ansprüchen, **dadurch gekennzeichnet, dass** sie weiterhin linguale Stimulatoren aufweist, wobei die genannte Vorrichtung (1) zumindest einen lingualen Stimulator (13) aufweist, der bevorzugt durch ein Agglomerat kleiner Kügelchen repräsentiert wird, die bevorzugt auf der Oberfläche (11) des flachen oder konkaven Endes (3") des gekrümmten Teils (3) zu platzieren sind.

6. Neurophysiologische Stimulationsvorrichtung (1) gemäß den vorangehenden Ansprüchen, **dadurch gekennzeichnet, dass** der Ambossteil (2) eine Länge, die zwischen 2 cm und 6 cm und bevorzugt 3 cm beträgt, eine Breite, die zwischen 0,2 cm und 3 cm und nahe des ersten Endes (2') und des zweiten Endes (2") bevorzugt 2 cm beträgt und die in der zentralen Zone bevorzugt 1,5 cm beträgt, eine Dicke, die zwischen 0,3 cm und 1 cm und die in der zentralen Zone des genannten Ambossteils (2) bevorzugt 0,4 cm beträgt und an dem ersten Ende (2') und dem zweiten Ende (2") bevorzugt 0,7 cm beträgt, aufweist, dadurch, dass der gekrümmte Teil (3) in der Nähe des konvexen Endes (3') und des flachen oder konkaven Endes (3") eine Dicke zwischen 0,2 cm und 3 cm und bevorzugt 1,5 cm aufweist, ein Krümmungsradius, wenn das genannte flache oder konkave Ende (3") gekrümmt ist, an seinem flachen oder konkaven Ende (3") einen Krümmungsradius zwischen 0,5 cm und 1,5 cm und bevorzugt 1 cm beträgt, und an dem konvexen Ende (3') einen Krümmungsradius zwischen 0,5 cm und 3 cm und bevorzugt 2 cm aufweist, und dadurch, dass der Einschnitt (4) eine Tiefe aufweist, die zwischen 0,1 cm und 0,8 cm variiert und bevorzugt 0,3 cm beträgt.

7. Neurophysiologische Stimulationsvorrichtung gemäß den vorangehenden Ansprüchen, **dadurch gekennzeichnet, dass** sie weiterhin mit Substanzen erhalten wird, die dazu ausgebildet sind, die lingual-sensorische Stimulation zu verbessern, wobei die genannte Vorrichtung (1) optional mit essbaren Substanzen, die verschiedene Geschmacksrichtungen an die genannte Vorrichtung (1) und bevorzugt fruchtige Geschmacksrichtungen verleihen, erhalten wird.

8. Vorrichtung (1) für durch Gesichtsentspannung hervorgerufene neurophysiologische Stimulation gemäß den vorangehenden Ansprüchen, **dadurch gekennzeichnet, dass** das palatale Element (5) für die palatale sensorische Stimulation, gemessen von dem ersten Ende (5') zu dem zweiten Ende (5") eine Länge, die zwischen 3 cm und 7 cm und bevorzugt 3,5 cm beträgt, einen Krümmungsradius, der zwischen 1 cm und 3 cm und bevorzugt 2 cm beträgt, und eine Breite, die zwischen 1 cm und 6 cm und die an den lateralen Vorsprüngen (5'''), die an dem ersten Ende des palatalen Elements (5') platziert sind, bevorzugt 2 cm beträgt, und an dem zweiten Ende des palatalen Elements (5") bevorzugt 4 cm beträgt, aufweist

## Revendications

1. Dispositif (1) pour une stimulation neurophysiologique induite par une relaxation faciale, comprenant : une partie en forme d'enclume (2) qui a un profil d'enclume et qui présente une première extrémité (2') et une seconde extrémité (2") ; une partie courbe (3) qui a un profil arqué et qui présente une extrémité convexe (3') et une extrémité plate ou concave (3"), une rainure (4) qui est prévue sur ladite partie courbe (3) et qui suit la même courbe que celle-ci, la partie en forme d'enclume (2) et la partie courbe (3) formant un seul corps, la partie en forme d'enclume (2) et la partie courbe (3) étant reliées solidement grâce au fait que la seconde extrémité (2") de la partie en forme d'enclume (2) est reliée à l'extrémité convexe (3') de la partie courbe (3), ladite partie courbe (3) étant apte à représenter la zone d'occlusion de la bouche de l'utilisateur, ladite partie courbe (3) présentant la rainure (4) qui s'étend d'une canine à l'autre quand le dispositif (1) est appliqué sur le sujet utilisateur du dispositif (1), l'extrémité plate ou concave (3") s'étendant à l'intérieur de la bouche, par rapport aux dents du sujet, tandis que l'extrémité convexe (3') s'étend à l'extérieur des dents de l'utilisateur, la rainure (4) étant délimitée par l'extrémité convexe (3') et par l'extrémité plate ou concave (3"), ladite rainure (4) étant prévue sur la première surface (1') et/ou sur la seconde surface (1") du dispositif (1), et de préférence au moins sur la surface impliquant l'occlusion dentale des canines de l'arcade dentaire supérieure, et la partie en forme d'enclume (2) étant apte à représenter la poignée du dispositif (1), ladite partie en forme d'enclume (2) s'étendant à l'extérieur de la bouche de l'utilisateur au moment de son application,
le dispositif (1) étant **caractérisé en ce qu'**il comprend un élément palatal (5) qui présente une première extrémité d'élément palatal (5') et une seconde extrémité d'élément palatal (5"), ledit élément palatal (5) étant adapté pour une stimulation sensorielle de l'arcade de l'avant du palais, la seconde extrémité (5") de l'élément palatal étant en contact, lors de l'utilisation, avec la voûte palatine, et l'élément palatal (5) étant conçu pour être assemblé et verrouillé de manière réversible sur le dispositif (1) au niveau de sa partie courbe (3), ladite partie courbe (3) présentant une fente (6) sur son extrémité plate ou concave (3"), cette fente étant apte à permettre le passage de l'élément palatal (5) d'une surface du dispositif (1) à l'autre, le dispositif (1) présentant la première surface (1') et la seconde surface (1"), et l'élément palatal (5) ayant sa première extrémité (5') pourvue de saillies latérales (5''') aptes à permettre l'introduction manuelle et le verrouillage de l'élément palatal (5) à travers la fente (6) et en même temps apte à empêcher le détachement involontaire de l'élément palatal (5) du dispositif (1), les saillies latérales (5''') conférant une plus grande largeur à la première extrémité d'élément palatal (5') par rapport à celle de la fente (6).

2. Dispositif (1) pour une stimulation neurophysiologique induite par une relaxation faciale selon la revendication précédente, **caractérisé en ce que** l'élément palatal (5) pour la stimulation sensorielle du palais comprend également au moins une paire de pattes flexibles (7) qui sont placées le long des grands côtés de l'élément palatal (5) et qui sont aptes à ajuster l'extension de ce dernier le long du palais du sujet utilisateur du dispositif (1).

3. Dispositif de stimulation neurophysiologique (1) selon les revendications précédentes, **caractérisé en ce qu'**il comprend également des systèmes pour administrer des principes ou substances actifs à action phytothérapeutique, le dispositif (1) comportant la partie en forme d'enclume (2) creuse et apte à contenir la substance phytothérapeutique, et au moins un orifice de pulvérisation (8) situé de préférence près de la première extrémité (2') de ladite partie en forme d'enclume (2) et adapté pour l'écoulement de la substance, la partie en forme d'enclume (2) se composant d'un polymère amorphe qui est compressible manuellement, et/ou **en ce qu'**il comprend des systèmes à libération lente de principes actifs ou de substances phytothérapeutiques, le dispositif (1) comprenant à titre optionnel une membrane (9), adsorbante pour les principes actifs et/ou pour les substances à action phytothérapeutique, sur la surface de la partie en forme d'enclume (2), et apte à retenir lesdites substances après immersion de ladite partie en forme d'enclume (2) dans un fluide phytothérapeutique, ou un fluide contenant des principes actifs, et à libérer lentement la substance phytothérapeutique ou les principes actifs à proximité des récepteurs olfactifs du sujet quand il/elle utilise le dispositif (1).

4. Dispositif de stimulation neurophysiologique (1) selon les revendications précédentes, **caractérisé en ce qu'**il comprend également un système pour administrer des substances et de préférence pour administrer des principes actifs ou des substances phytothérapeutiques à administrer directement et par voie orale au sujet, le dispositif (1) comprenant au moins un conduit (10) apte à contenir la substance fluide, ce conduit étant dirigé intérieurement et longitudinalement par rapport au dispositif (1), de la partie en forme d'enclume (2) jusqu'à la partie courbe (3), et au moins un orifice (12) placé sur l'extrémité (3") de la partie (3), l'extrémité convexe (3") présentant la surface (11) sur laquelle l'orifice (12) est placé de préférence, et la partie en forme d'enclume (2) se composant d'un matériau compressible, de manière à induire la compression indirecte du conduit (10) qui contient le fluide à administrer et qui a été introduit précédemment à l'aide de moyens et d'opérations d'un type connu.

5. Dispositif de stimulation neurophysiologique (1) selon les revendications précédentes, **caractérisé en ce qu'**il comprend également des stimulateurs linguaux, le dispositif (1) comprenant au moins un stimulateur lingual (13), représenté de préférence par un agglomérat de sphérules, à placer de préférence sur la surface (11) de l'extrémité plate ou concave (3") de la partie courbe (3).

6. Dispositif de stimulation neurophysiologique (1) selon les revendications précédentes, **caractérisé en ce que** la partie en forme d'enclume (2) a une longueur comprise entre 2 cm et 6 cm, et de préférence de 3 cm, une largeur comprise entre 0,2 cm et 3 cm, et de préférence de 2 cm à proximité de la première extrémité (2') et de la seconde extrémité (2") et de préférence de 1,5 cm dans la zone centrale, une épaisseur comprise entre 0,3 cm et 1 cm et de préférence de 0,4 cm dans la zone centrale de la partie en forme d'enclume (2) et de préférence de 0,7 cm à la première extrémité (2') et à la seconde extrémité (2"), **en ce que** la partie courbe (3) a une épaisseur, à proximité de l'extrémité convexe (3') et de l'extrémité plate ou concave (3"), comprise entre 0,2 cm et 3 cm et de préférence de 1,5 cm, un rayon de courbure compris entre 0,5 cm et 1,5 cm et de préférence de 1 cm à ses extrémités plates ou concaves (3") quand ladite extrémité plate ou concave (3") est courbe, et un rayon de courbure compris entre 0,5 cm et 3 cm et de préférence de 2 cm à l'extrémité convexe (3'), et **en ce que** la rainure (4) a une profondeur qui varie entre 0,1 cm et 0,8 cm et de préférence de 0,3 cm.

7. Dispositif de stimulation neurophysiologique (1) selon les revendications précédentes, **caractérisé en ce qu'**il est également obtenu avec des substances aptes à augmenter la stimulation sensorielle linguale, le dispositif (1) étant obtenu à titre optionnel avec des substances comestibles qui confèrent différents goûts au dispositif (1), et de préférence des arômes fruités.

8. Dispositif (1) pour une stimulation neurophysiologique induite par une relaxation faciale selon les revendications précédentes, **caractérisé en ce que** l'élément palatal (5) pour la stimulation sensorielle palatale a une longueur comprise entre 3 cm et 7 cm et de préférence de 3,5 cm, mesurée de la première extrémité (5') jusqu'à la seconde extrémité (5"), un rayon de courbure compris entre 1 cm et 3 cm et de préférence de 2 cm, et une largeur comprise entre 1 cm et 6 cm et de préférence de 2 cm au niveau des saillies latérales (5''') placées à la première extrémité d'élément palatal (5'), et de préférence de 4 cm à la seconde extrémité d'élément palatal (5").
